# EUROPEAN PATENT APPLICATION

(11) **EP 2 570 151 A1**
(43) Date of publication of application: **20.03.2013**
(21) Application number: 12184668.7
(22) Date of filing: 17.09.2012
(51) Int. Cl.: A61M 35/00, B05B 15/12, E04H 15/20

(54) **Inflatable structure**

(30) Priority: 16.09.2011 GB 201116056
(71) Applicant: Sunless Solutions Limited, Wigmore, Kent ME8 0PW (GB)
(72) Inventor: Young, Lisa, Wigmore, Kent ME8 0PW (GB)
(74) Representative: Bridle, Andrew Barry

(57) **Abstract**

An inflatable structure for use as a spray tan booth, the structure including an inflatable support assembly which defines a pair of opposed sides and a front, the inflatable support assembly including a valve which is connectable to a source of compressed air and having an erect configuration and a collapsed configuration; a pair of opposed side wall elements supported by the support assembly and a floor element located between the side wall elements, wherein, in the erect configuration, the front defines an opening which provides unimpeded access into the structure, and wherein the inflatable support assembly comprises a material which is gas permeable, whereby a constant supply of compressed air is necessary to maintain the assembly in an erect configuration.

## Description

The present invention relates to an inflatable structure, and, in particular, to an inflatable structure for use as a spray tan booth.

As research over the last several years has confirmed a link between exposure to the sun and skin conditions, including cancer, alternative ways of artificially giving the skin a "tanned" look have become more popular. This includes a process known as "spray tanning", where a subject is sprayed with a cosmetic preparation that tints the skin to provide a tanned look.

Typically, for the application of a spray tan, the subject stands in a spray tan booth to be sprayed with the cosmetic preparation to avoid the cosmetic preparation contacting and possibly damaging surfaces or objects which are not intended to be sprayed. The object for the booth is to catch the overspray and retain it within the interior of the booth. It will be appreciated that the terms "cosmetic preparation", "spray tan preparation" and the like are intended to refer to a formulation adapted to be sprayed onto the skin of a subject to impart thereto a coloured tint or tanned effect.

It is an aim of the present invention to provide an inflatable structure that may be used as a portable spray tan booth which is light and compact when in a deflated state, to allow for ease of portability, but which can be quickly and easily inflated for use as a spray tan booth.

According to a first aspect, the present invention provides an inflatable structure for use as a spray tan booth, the structure including an inflatable support assembly which defines a pair of opposed sides and a front, the inflatable support assembly including a valve which is connectable to a source of compressed air and having an erect configuration and a collapsed configuration; a pair of opposed side wall elements supported by the support assembly and a floor element located between the side wall elements, wherein, in the erect configuration, the front defines an opening which provides unimpeded access into the structure, and wherein the inflatable support assembly comprises a material which is gas permeable, whereby a constant supply of compressed air is necessary to maintain the assembly in an erect configuration.

The skilled person will appreciate that the booth must have a height when inflated that enables a subject to stand within it to be sprayed with the cosmetic preparation in order for it to be useful as a spray tan booth. Furthermore, spray tan booths are typically used within a building, so the inflatable structure is suitably intended for use inside a building.

By the term "unimpeded access" it is meant that access to the front is not impeded by any element of the structure. Thus, the front of the structure defines the opening through which a user can pass wherein no panels or elements of the structure are located in, or are capable of covering, the opening. The front of the structure may include one or more panels at the side of or above the opening, provided that the opening itself is free from all such panels or elements. It is important that the opening provides a person applying the cosmetic formulation with complete access to a subject located within the structure, so that the cosmetic formulation can be applied evenly to the entire body or the desired portions of the body. Accordingly, the opening must be sufficiently sized to achieve this object.

The inflatable support assembly suitably also defines a rear of the structure and supports a rear wall element in addition to the side wall elements. Accordingly, the opposed side wall elements may each contact a respective side of the rear wall element or rear support element.

However, where no rear wall element is present, for example, where the two side wall elements curve around to form mutually adjacent portions at the rear of the structure, the inflatable support assembly may include a rear support element from which both side wall elements are supported.

The skilled person will appreciate that the side wall elements may be separate components or may be defined by a single component which extends from one side of the opening, around the rear of the structure to the other side of the opening.

The inflatable support assembly, together with the side wall elements and optional rear wall element define an enclosure having opposed walls and an open front which together form a structure that can be used as a spray booth. The unimpeded opening allows the operator administering the cosmetic preparation to have full access to the body of the subject, from their feet to their head.

It is usual for the applicator of the cosmetic tanning preparation to be a spray gun which operates using a source of compressed air, typically from an air compressor. By including a valve which is connectable to a source of compressed air, such as an air compressor, the inflatable structure can be inflated via the air compressor. In an embodiment, the source of compressed air includes at least two outputs, wherein one of the outputs may provide compressed air to the inflatable structure and another output may provide compressed air to a spray gun for applying the tanning preparation. In the latter embodiment, the compressor itself may have two or more outputs or it may include an output conduit which is branched to provide the requisite outputs. Suitably, the compressor may include one output and a bifurcated conduit (such as a hose) having a single input and two separate outputs. Thus, the same air compressor can be used to inflate the structure, to maintain it in an erect configuration and to apply the cosmetic preparation. Accordingly, no additional equipment is necessary to erect the structure, as the air compressor is part of the equipment required to apply the cosmetic preparation.

In an alternative embodiment, two separate compressors may be supplied: one to inflate the structure and the other to provide a compressed air supply to the spray gun.

According to the invention, the inflatable support assembly is not airtight and requires a constant supply of compressed air to maintain it in an erect configuration. Accordingly, removal of the compressed air source causes the support assembly to collapse, allowing it to be arranged in a compact storage/transportation configuration. The use of a gas permeable material enables the structure to be very rapidly inflated and very rapidly deflated. It also allows for the structure to be made from a machine-washable fabric or material. This is important, given that the structure will necessarily become contaminated with spray tan preparation in use. It is much easier for a user simply to place the structure in a conventional washing machine to clean it, rather than trying to clean it in an erect configuration and allow it to dry prior to deflation and configuration for storage.

In an embodiment of the invention, the inflatable support assembly includes a front support element, a rear support element and at least one intermediate support element located between the front support element and the rear support element. In this embodiment, the front support element defines the unimpeded opening, the rear support element supports a rear wall element and the or each intermediate support element spaces the front support element from the rear support element to define the pair of opposed sides. A portion of the front support element, a portion of the rear support element and a portion of the or each intermediate support element together support a first side wall element and opposing portions of the front support element, rear support element and intermediate support element(s) support a second side wall element to provide opposing side wall elements.

In a further embodiment of the invention, the front support element, the rear support element and the at least one intermediate support element are in fluid communication with each other. In this arrangement, only one valve is necessary to inflate the entire structure, as the compressed air introduced into the valve is able to flow between the front, rear and intermediate support elements.

In a still further embodiment of the invention, the front support element and the rear support element are both in the form of an inverted U. The inverted U shape, when inflated, causes the floor element to be drawn taught as the arms of the U are urged outwards by the pressure of the air within them. In a yet further embodiment of the invention the or one of the intermediate support elements connects the apex of the front support element to the apex of the rear support element. Such an arrangement avoids the need to provide a separate roof element to enclose the structure, as the side wall elements and the rear wall element all extend to the apex of the structure.

However, an alternative embodiment, where an intermediate support is not provided at the apex of the inverted U shaped front and rear support elements, is within the scope of the invention. In this embodiment, a roof element supported by two or more intermediate support elements may be provided.

The inflatable support assembly may include a first base intermediate support which connects a first end of the front support element to a first end of the rear support element, and a second base intermediate support which connects a second end of the front support element to a second end of the rear support element. According to this embodiment, the front support element is spaced from the rear support element by at least two intermediate support elements, which forms a more rigid structure when inflated.

Where the support assembly includes inverted U shaped front and rear support elements, an intermediate support element which connect the apices of the front and rear support elements and first and second base intermediate support elements, each side wall element is supported on four sides (a portion of the front support, a portion of the rear support, one of the base intermediate supports and apex intermediate support) and is able to conform accurately to the sides as defined by the inflatable support assembly. Such an arrangement forms a stable structure when inflated.

In an embodiment of the invention, the floor element connects the first base intermediate support, the second base intermediate support and the rear wall element. This arrangement urges the floor element taught upon inflation and provides a flat, planar floor surface that minimises the risk of presenting a trip hazard.

The floor element may extend outwardly beyond the front defined by the support assembly. This arrangement may provide protection against overspray contacting flooring forwards of the booth via the opening.

Elements of the structure or portions of the elements may be translucent or transparent to permit light to enter the structure, thereby illuminating a subject therein. Illumination of a subject within the structure makes it easier for the person applying the cosmetic formulation to apply it evenly without gaps or over-application.

In a further embodiment of the invention, one of the wall elements, such as the rear wall element, includes a flap. This allows for an extractor fan to be located through the flap of the structure to draw contaminated air out of the structure. Thus, the flap may be opened to expose an opening in the relevant wall element through which an inlet port of an extractor may be located or it may be closed to cover the opening and prevent any of the cosmetic preparation exiting the structure via the wall element. The flap may include one part of a releasable closure such that it can be maintained in a closed position if desired. Typically, the releasable closure is a two-part closure. Thus, one part of the closure may be carried by the flap and the other part of the closure may be carried by the wall element, suitably adjacent the flap. For example, the flap may include one part of a zip or a hook and eye-type closure such that the flap can be maintained in the closed position if no extractor fan is to be used or it may be opened by release of the closure, e.g. by unzipping the zip or urging apart the hook and eye portions.

As structures suitable for use as spray tan booths tend to get dirty relatively quickly, as a nature of their use, the components of the structure are suitably made from washable materials. In an embodiment of the invention, the structure, when deflated is capable of being located within a washing machine, such as a domestic washing machine. Furthermore, the structure may be made from materials which are adapted to be washed within a washing machine. Thus, the structure may be made from fabric materials which are resilient to the temperatures, detergent compositions and conditions experienced within domestic washing machines, i.e. the structure is machine washable.

According to a second aspect of the invention, there is provided an inflatable spray tan booth including an inflatable support assembly which defines a pair of opposed sides and a front, the inflatable support assembly including a valve which is connectable to a source of compressed air and having an erect configuration and a collapsed configuration; a pair of opposed side wall elements supported by the support assembly and a floor element located between the side wall elements, wherein, in the erect configuration, the front defines an opening which provides unimpeded access into the structure, and wherein the inflatable support assembly comprises a material which is gas permeable, whereby a constant supply of compressed air is necessary to maintain the assembly in an erect configuration.

The features described and defined in connection with embodiments of the inflatable structure according to the first aspect of the invention are applicable to corresponding embodiments of the inflatable spray tan booth according to the second aspect of the invention.

According to a third aspect of the invention, there is provided a spray tan kit including an inflatable structure as defined anywhere hereinabove, an air compressor and a spray gun, wherein both the spray gun and the inflatable structure are connectable to the compressor.

In one embodiment of the invention, two outputs are provided from the air compressor and both the inflatable structure and the spray gun may be connected to the air compressor simultaneously. In this embodiment, the two outputs may be provided by the compressor itself or the compressor may include a single output and a hose which includes two outputs.

In embodiments where the compressor includes two outputs, it suitably further includes a controller to control the flow of compressed air through each of the outputs. Thus, the flow of compressed air though each of the outputs may be varied as desired. It will be appreciated that a relatively high flow of compressed air is desirable when inflating the structure from its collapsed configuration. However, when in its erect configuration a relatively smaller flow of compressed air is needed to maintain the structure erect.

Accordingly, in an embodiment of the invention, the controller includes a first configuration in which substantially all of the compressed air generated by the compressor is directed to the first output and a second configuration in which compressed air is directed to both outputs with a greater amount of the compressed air being directed to the second output compared with the first output. Thus, the first output may be connected to the valve of the structure and the second output may be connected to the spray gun. In this way, the controller can provide an inflation setting in which substantially all of the compressed air is directed to the structure, and a spray setting in which a maintenance flow of compressed air is directed to the structure in order to maintain it in an erect configuration and a greater flow of compressed air is directed to the spray gun for application of the spray tan preparation to a subject.

According to a fourth aspect of the invention, there is provided a spray tan kit including an inflatable structure as defined anywhere herein; a pair of compressors; and a spray gun, wherein the inflatable structure is connectable to a first of the compressors and the spray gun is connectable to the second compressor.

According to a fifth aspect of the invention, there is provided a method of applying a spray tan to a subject, the method comprising the steps:
i. connecting an inflatable structure as described anywhere herein to a first source of compressed air;
ii. inflating the inflatable structure with compressed air;
iii. maintaining the inflatable structure in an erect configuration by maintaining a supply of compressed air to it;
iv. locating the subject within the inflatable structure;
v. connecting a spray gun containing a spray tan preparation to a second source of compressed air; and
vi. spraying the spray tan preparation onto the skin of the subject within the inflatable structure.

In an embodiment of the invention according to the fifth aspect, the source of compressed air is an air compressor. The first and second sources of compressed air may be provided by a single compressor or they may be provided by two separate compressors.

Thus, the method may include the step of connecting the inflatable structure to a first output from the compressed air source and connecting the spray gun to a second output from the compressed air source.

The skilled person will appreciate that the features described and defined in connection with the aspects of the invention and the embodiments thereof may be combined in any combination, regardless of whether the specific combination is expressly mentioned herein. Thus, all such combinations are considered to be made available to the skilled person.

An embodiment of the invention will now be described, by way of example only, with reference to the accompanying drawings in which:
Figure 1 is a perspective view of an inflatable structure according to the invention;
Figure 2 is a side elevational view of the structure shown in Figure 1; and
Figure 3 is a front elevational view of the structure.

For the avoidance of doubt, the skilled person will appreciate that in this specification, the terms "up", "down", "front", "rear", "upper", "lower", "width", etc. refer to the orientation of the components as found in the example when installed for normal use as shown in the Figures.

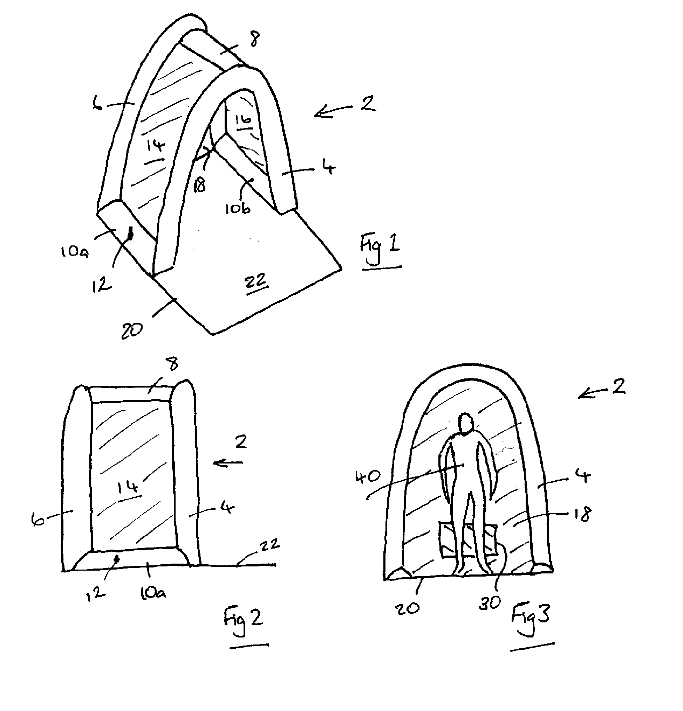

An inflatable structure in the form of an inflatable spray tan booth 2 is shown in Figures 1, 2 and 3. The booth 2 comprises a support assembly formed from a front inflatable support tube 4, a rear inflatable support tube 6, a top inflatable support tube 8 and a pair of base inflatable support tubes 10a, 10b. The front support tube 4 and the rear support tube 6 are in the form of inverted U shaped tubes, with the apices of the tubes 4, 6 joined via the top support tube 8. The respective arms of the tubes 4, 6 are joined via the base support tubes 10a, 10b. All of the support tubes 4, 6, 8, 10a, 10b are interconnected such that air can flow throughout the structure assembly and the support assembly can be inflated via a single air inlet valve 12 located in the base inflatable support tube 10a.

The support tubes 4, 6, 8, 10a, 10b are all formed from a gas permeable, machine washable material.

The support assembly defined by the support tubes 4, 6, 8, 10a, 10b supports wall elements or panels which define an interior space. More specifically, the top support tube 8, the base support tube 10a and the portions of the front support tube 4 and the rear support tube 6 located between them support a first (or left hand side) wall panel 14. Similarly, the other base support tube 10b, the top support tube 8 and the portions of the front support tube 4 and the rear support tube 6 located between them support a second (or right hand side) wall panel 16.

The rear support tube 6 also supports a rear wall panel 18, which entirely covers the rear wall defined by the rear support tube 6. The rear wall panel 18 includes a flap 30 (shown in Figure 3), which may be opened to allow an air extractor fan to be used in conjunction with the booth 2 or may be left closed to prevent escape of a tanning preparation from the booth 2.

Located between the two opposed base support tubes 10a, 10b and the bottom edge of the rear wall panel 18 is a floor sheet 20. As shown in Figures 1 and 2, a portion 22 of the floor sheet 20 extends beyond the front of the support assembly as defined by the front support tube 4.

As shown in Figure 3, the front of the booth 2 as defined by the front support tube 4 is open and unimpeded.

In use, the deflated or collapsed booth 2 is connected to a first outlet of an air compressor (not shown) via the inlet valve 12. Compressed air from the first outlet of the compressor is forced into the base support tube 10a and from there into the front support tube 4 and the rear support tube 6. Finally, the air enters and inflates the top support tube 8 and the other base support tube 10b. As the front support tube 4 inflates, the opposed arms of the U-shaped tube are urged apart, which pulls taught the portion of the floor sheet 20 between them. Similarly, as the rear support tube 6 inflates, the opposed arms of the U-shaped tube are urged apart, pulling taught the portion of the floor sheet 20 located between them. In addition, the inflation of the rear wall support tube 6 pulls taught the rear wall panel 18.

The inflated base support tube 10a, 10b separate the front support tube 4 and the rear support tube 6 and has the effect of pulling taught the floor sheet in a second, perpendicular direction. Thus, the forces exerted in a first (lengthwise) direction on the floor sheet 20 by the base support tubes 10a, 10b and the forces exerted in a second (widthwise) direction on the floor sheet 20 by the arms of the U-shaped front and rear support tubes 4, 6, pulls the floor sheet 20 fully taught and causes it to adopt a substantially planar configuration.

The top support tube 8 separates the apices of the front and rear support tubes 4, 6 and pulls taught the side wall panels 14, 16.

In this configuration, the support assembly and thus the booth 2 is said to be in an erect configuration. Once fully inflated and in an erect configuration, with the support assembly containing a desired air pressure and with the wall panels 14, 16, 18 and floor sheet 20 pulled taught, the air compressor provides a maintenance flow of compressed air to maintain the support assembly in the erect configuration. This is required because the support assembly is constantly losing air through the material from which it is formed.

A second outlet of the air compressor is then connected to a spray gun (not shown) and is used to supply compressed air to the spray gun, which is used to apply the cosmetic preparation to a subject 40 standing within the fully inflated booth 2 (see Figure 3). The absence of any impediment in the opening as defined by the front support tube 4 means that the entire body of the subject 40 is readily accessible to the person applying the spray tan preparation. Accordingly, an even application of the spray tan preparation is possible.

The side wall panels 14, 16 and the rear wall panel 18 prevents any excess spray tan preparation from unintentionally exiting the booth via the sides or the rear and possibly damaging any items located outside of the booth 2, such as floor coverings and/or furniture. The projecting portion 22 of the floor sheet 20 prevents excess spray tan preparation contacting a floor covering forward of the booth 2.

The booth 2 is deflated by removing the maintenance supply of compressed air from the compressor. The gas permeable nature of the support assembly material allows the compressed air located therein to diffuse through the material very quickly, resulting in a speedy and complete deflation of the support assembly and permitting the structure to be placed in a storage or transport configuration.

In order to provide two outputs from the compressor, it includes an output hose having a Y-shaped adaptor to provide the two separate outputs from the output hose. Thus, the output hose is bifurcated. The inflatable structure is connected to one of the two outputs from the output hose via the inlet valve 12 and compressed air is supplied to the inflatable support structure all the time the booth is in use. The spray gun is connected to the second output of the output hose and used as normal.

The output hose includes an air flow controller which controls the flow of compressed air through its two outputs. The flow controller has a first configuration in which substantially all of the compressed air flow is directed to the first output in order to inflate the support assembly into its erect configuration. Once in its erect configuration, the controller may be moved to a second configuration in which a maintenance flow of compressed air is provided to the first output and a greater flow of compressed air is directed to the second output. In this way, the compressor is able to maintain the support assembly in its erect configuration and it is able to provide a suitable supply of compressed air to the spray gun to enable a spray tan preparation to be sprayed from it.

Air compressors, bifurcated hoses and air flow controllers are all well known and it is not necessary to provide further details of these components.

## Claims

1. An inflatable structure for use as a spray tan booth, the structure including an inflatable support assembly which defines a pair of opposed sides and a front, the inflatable support assembly including a valve which is connectable to a source of compressed air and having an erect configuration and a collapsed configuration; a pair of opposed side wall elements supported by the support assembly and a floor element located between the side wall elements, wherein, in the erect configuration, the front defines an opening which provides unimpeded access into the structure, and wherein the inflatable support assembly comprises a material which is gas permeable, whereby a constant supply of compressed air is necessary to maintain the assembly in an erect configuration.

2. An inflatable structure according to Claim 1, wherein the inflatable support assembly includes a front support element, a rear support element and at least one intermediate support element located between the front support element and the rear support element such that the front support element is spaced from the rear support element.

3. An inflatable structure according to Claim 2, wherein the front support element, the rear support element and the at least one intermediate support element are in fluid communication with each other.

4. An inflatable structure according to Claim 2 or Claim 3, wherein the front support element and the rear support element are both in the form of an inverted U and the or one of the intermediate support elements connects the apex of the front support element to the apex of the rear support element.

5. An inflatable structure according to any of Claims 2 to 4, wherein the inflatable support assembly includes a first base intermediate support which connects a first end of the front support element to a first end of the rear support element, and a second base intermediate support which connects a second end of the front support element to a second end of the rear support element.

6. An inflatable structure according to Claim 5, wherein the floor element connects the first base intermediate support, the second base intermediate support and a rear wall element.

7. An inflatable structure according to any preceding claim, wherein the floor element extends outwardly beyond the front defined by the support assembly.

8. An inflatable structure according to any preceding claim, wherein one of the wall elements includes a flap.

9. A spray tan kit including an inflatable structure according to any of Claims 1 to 8, an air compressor and a spray gun, wherein both the spray gun and the inflatable structure are connectable to the compressor.

10. A spray tan kit according to Claim 9, wherein the air compressor includes two outputs, the first of which is adapted for connection to the inflatable structure and the second of which is adapted for connection to the spray gun.

11. A spray tan kit according to Claim 10, wherein the compressor includes a controller capable of controlling the flow of compressed air through the outputs.

12. A spray tan kit according to Claim 11, wherein the controller includes a first configuration in which substantially all of the compressed air generated by the compressor is directed to the first output and a second configuration in which compressed air is directed to both outputs with a greater amount of the compressed air being directed to the second output compared with the first output.

13. A method of applying a spray tan to a subject, the method comprising the steps:
i. connecting an inflatable structure according to any of claims 1 to 8 to a source of compressed air;
ii. inflating the inflatable structure with compressed air;
iii. maintaining the inflatable structure in an erect configuration by maintaining a supply of compressed air to the structure;
iv. locating the subject within the inflatable structure;
v. connecting a spray gun containing a spray tan preparation to the source or a second source of compressed air; and
vi. spraying the spray tan preparation onto the skin of the subject within the inflatable structure.

14. A method according to Claim 13, wherein the or each source of compressed air is an air compressor.
